(19) Europäisches Patentamt / European Patent Office / Office européen des brevets

(11) **EP 2 586 404 A1**

(12) **EUROPÄISCHE PATENTANMELDUNG**

(43) Veröffentlichungstag:
01.05.2013 Patentblatt 2013/18

(51) Int Cl.:
*A61F 2/82* (2013.01) *A61B 5/00* (2006.01)

(21) Anmeldenummer: 12188615.4

(22) Anmeldetag: 16.10.2012

(84) Benannte Vertragsstaaten:
AL AT BE BG CH CY CZ DE DK EE ES FI FR GB GR HR HU IE IS IT LI LT LU LV MC MK MT NL NO PL PT RO RS SE SI SK SM TR
Benannte Erstreckungsstaaten:
BA ME

(30) Priorität: 27.10.2011 US 201161551941 P

(71) Anmelder: Biotronik AG
8180 Bülach (CH)

(72) Erfinder:
• Wittchow, Eric
91710 Gunzenhausen (DE)
• Skerl, Olaf
18209 Bad Doberan (DE)
• Czygan, Gerald
91054 Buckenhof (DE)

(74) Vertreter: Lindner-Vogt, Karin L.
Biotronik SE & Co. KG
Corporate Intellectual Properties
Woermannkehre 1
12359 Berlin (DE)

(54) **Implantat**

(57) Die vorliegende Erfindung beschreibt ein Implantat (100) mit einem vorzugsweise hohlzylinderförmigen Grundkörper (101) und mit einer Einrichtung, die zur Messung des Grads der Endothelialisierung dient und die an und/oder im Grundkörper angeordnet ist, wobei die Einrichtung einen akustischen Resonator (103) und/oder einen elektrischen Resonator (121) aufweist. Die Erfindung betrifft ferner ein System aus einem Katheter und einem derartigen Implantat.

**FIG. 1**

EP 2 586 404 A1

**Beschreibung**

Die vorliegende Erfindung betrifft ein Implantat, insbesondere eine intraluminale Endoprothese, mit einem vorzugsweise hohlzylindrischen Grundkörper sowie ein System aus einem Katheter und einem derartigen Implantat.

Medizinische Endoprothesen oder Implantate für die unterschiedlichsten Anwendungen sind in großer Vielfalt aus dem Stand der Technik bekannt. Als Implantate im Sinne der vorliegenden Erfindung sind endovaskuläre Prothesen oder sonstige Endoprothesen, beispielsweise Stents (Gefäß-Stent (einschließlich des Herzens und Herzklappenstents), Gallengang-Stent, Mitral-Stent), Endoprothesen zum Verschließen von persitierenden Foramen ovale (PFO), Pulmonalklappenstents (pulmonary valve stent), Endoprothesen zum Verschließen eines ASD (Vorhofscheidewanddefekt, atrial septal defect) sowie Prothesen im Bereich des Hart- und Weichgewebes zu verstehen.

Heutzutage werden als Implantate besonders häufig Stents eingesetzt, die zur Behandlung von Stenosen (Gefäßverengungen) dienen. Sie weisen einen meist durchbrochenen in der Regel rohrförmigen oder hohlzylinderförmigen Grundkörper auf, der an beiden Längsenden offen ist. In vielen Fällen setzt sich der Grundkörper des Stents aus einzelnen Maschen zusammen, welche aus verschieden geformten Stegen (Struts), beispielsweise zick-zack-oder mäander-förmigen Stegen, gebildet werden. Eine derartige Endoprothese wird in der Regel mittels eines Katheters in das zu behandelnde Gefäß eingesetzt und dient dazu, das Gefäß über einen längeren Zeitraum (Monate bis Jahre) zu stützen. Durch den Einsatz von Stents können verengte Bereiche in den Gefäßen erweitert werden, so dass ein Lumengewinn resultiert. Durch den Einsatz von Stents oder anderen Implantaten kann zwar ein für den Therapieerfolg primär notwendiger optimaler Gefäßquerschnitt erreicht werden, allerdings initiiert die dauerhafte Anwesenheit eines Stents, der an sich einen Fremdkörper darstellt, eine Kaskade von mikrobiologischen Prozessen, die beispielsweise eine Entzündung (Inflammation) des behandelten Gefäßes oder eine nekrotische Gefäßveränderung begünstigen und/oder die zu einem allmählichen Zuwachsen des Stents durch Bildung von Plaques bzw. Koagulation von Körperflüssigkeit infolge einer Strömungsveränderung oder infolge eines Inflammations- oder Infektionsprozesses führen können.

Um Restenosen bzw. Inflammationen und Nekrosen zu vermeiden, können Stents oder andere Implantate mit einer Beschichtung von Medikamenten oder anderen pharmazeutisch aktiven Substanzen versehen werden, die beispielsweise eine antikoagulierende, eine antiproliferative oder eine antiinflammatorische Wirkung besitzen. Derartige Stents werden auch als Drug Eluting Stents (im Folgenden kurz: DES) bezeichnet.

Durch die Einführung von DES, welche beispielsweise Paclitaxel oder Sirolimus eluieren, konnte die Restenoserate gegenüber Stents ohne Beschichtung deutlich gesenkt werden. Dies führt zu Kosteneinsparungen, da die Notwendigkeit einer Revaskularisierung signifikant sinkt.

Die Begeisterung für die neuen Therapieoptionen wird aber durch eine unveränderte oder sogar leicht angestiegene Zahl von späten Thrombosen (im Folgenden kurz: LST) gebremst. Metaanalysen zeigen, dass die Therapie mit DES von einem steigenden Prozentsatz an LST begleitet wird. Bei Patienten mit besonders ungünstigen Voraussetzungen kann die LST-Rate bis auf 8,2% ansteigen. Derartige Thrombosen müssen jedoch möglichst verhindert werden, da diese häufig einen tödlichen Ausgang (20 bis 40%) haben, zu Myocardinfarkten führen können (50 bis 70%) und oft eine Revaskularisierung erfordern.

Derzeit diskutierte Theorien geben als Hauptursache für DES-Spätthrombosen neben einer selten auftretenden Hypersensitivität gegenüber einigen DES-Komponenten einen verzögerten Heilungsverlauf an, welcher sich in einer persistierenden Fibrinablagerung und unvollständigen Endothelialisierung äußert. Die nicht endothelialisierten Stentstreben können im zuletzt genannten Fall die Ursache für eine Thrombose bilden. Diese Hypothese beruht vor allem auf Autopsie-Studien, bei denen in sämtlichen Fällen von LST eine verzögerte Heilung beobachtet wurde. Aktuelle Richtlinien empfehlen daher bei Implantation eines DES eine mehrmonatige, duale Antikoagulationstherapie mittels Aspirin und Clopidogrel, wodurch sich die gesamte Therapie deutlich verteuert. Ferner entstehen durch eine solche Therapie auch Wechselwirkungen mit anderen Therapien, insbesondere mit operativen Eingriffen.

Die optimale Dauer einer solchen Antikoagulationstherapie ist derzeit nicht geklärt. Aufschluss hierüber könnte eine Verlaufskontrolle der Heilung geben. Es ist daher wünschenswert, den Verlauf den Endothelialisierung während einer DES-Therapie zu überwachen.

Aus der Druckschrift "Sensor to detect endothelialization on an active coronary stent", K. M Musick et al., BioMedical Engineering OnLine 2010, 9: 67 ist ein Stent bekannt, der im Stentgitter mindestens einen abragenden Arm aufweist. Dieser enthält ein PiezoElement mit einer Schicht aus Zinkoxid. Bei einer Bedeckung des abragenden Arms (Cantilever) mit Körperzellen (Endothelialisierung) ändert sich die Resonanzfrequenz des Arms und ermöglicht so eine Messung des Grads der Endothelialisierung in vivo. Die bekannte Lösung ist jedoch hinsichtlich ihrer Abmessungen nachteilig, da ein solcher Arm weder in das Gefäß hineinragen noch den Aufbau des Stentkörpers wesentlich verändern soll, da dieser sonst durch Änderung der laminaren Strömung Proliferation auslösen oder Thromben verursachen könnte. Ferner entsprechen die Funktionalität und Elektronik nicht den Anforderungen für eine Anwendung im Bereich der Implantate. Hinsichtlich der Funktionalität ist festzustellen, dass die Resonanzfrequenz eines in einer Flüssigkeit schwingenden Cantilevers einerseits von dessen Massebeladung und andererseits stark von den Eigenschaften der umgebenden Flüssigkeit (z.B. deren Viskosität, Strömungsgeschwindigkeit) beeinflusst wird. Beide Effekte können nicht voneinander getrennt werden, wodurch eine zuverlässige Bestimmung der Massebeladung nicht gewährleistet werden kann. Zur Elektronik wird in dem genannten Stand der Technik erläutert, dass der Sensor als aktiver Sensor ausgeführt wird, der zusätzlich zum eigentlichen Transducer elektronische Elemente zur Messwerterzeugung und zum Datentransfer beinhaltet. Die dafür notwendige Hilfsenergie soll ebenfalls drahtlos von außen eingespeist werden. Die dadurch bedingte Baugröße der Elektronik der Lösung nach dem Stand der Technik, welche zusätzlich zum eigentlichen Transducer erforderlich ist, ist nicht akzeptabel.

Die Aufgabe besteht somit darin, ein Implantat zu schaffen, mit welchem die Endothelialisierung überwacht werden kann, ohne dass die äußeren Abmessungen und die Struktur des Implantats wesentlich verändert werden müssen.

Die obige Aufgabe wird durch ein Implantat mit einer Einrichtung (im Folgenden auch als Mikrosensor bezeichnet) gelöst, die zur Messung des Grads der Endothelialisierung dient und die an und/oder im Grundkörper angeordnet ist, wobei die Einrichtung einen akustischen Resonator und/oder einen elektrischen Resonator aufweist.

Das erfindungsgemäße Implantat mit der Einrichtung zur Messung des Grads der Endothelialisierung, welche einen akustischen Resonator und/oder einen elektrischen Resonator aufweist, ermöglicht die Bestimmung des Grads der Belegung des Implantats mit Endothel auf einfache Weise. Bei verzögerter Endothelialisierung kann die Antikoagulationstherapie individuell verlängert werden, bis bei einer erneuten Messung die Gewebeabdeckung des Implantats vollständig ist. So kann die Gefahr einer LST deutlich verringert werden. Umgekehrt kann die Antikoagulationstherapie bei schneller Gewebeabdeckung des Stents früher als nach dem derzeitigen Vorgehen ohne Verlaufskontrolle beendet werden, so dass Therapiekosten gespart werden und unnötige Nebenwirkungen mit anderen Therapien nur über einen kürzeren Zeitraum auftreten können. Der Vorteil des erfindungsgemäßen Implantats besteht daher insbesondere darin, dass die Medikamentierung nach vollständiger Endothelialisierung beendet werden kann. Anders ausgedrückt kann durch die vorliegende Erfindung verhindert werden, dass die Antikoagulationstherapie vor vollständiger Endothelialisierung abgesetzt wird.

Es ist möglich, einen akustischen Resonator oder ein elektrischen Resonator heute so kleinbauend zu realisieren, dass der Mikrosensor die Abmessungen des Implantats nicht wesentlich verändert. Der Mikrosensor wird vorzugsweise in und/oder an der luminalen Seite des Implantats angeordnet.

Vorzugsweise ist der Mikrosensor als rein passiver Sensor ausgeführt, der keine zusätzlichen elektronischen Einrichtungen enthält und für seine Funktion keine Hilfsenergie benötigt. Auch hierdurch ist eine extreme Miniaturisierung möglich.

Im Rahmen der vorliegenden Erfindung wird unter Grad der Endothelialisierung der Anteil der Gewebeabdeckung auf dem Sensor verstanden, welche sich nach dem Einsetzen des Implantats auf der Oberfläche desselben bildet. Wenn die Oberfläche des Implantats vollständig mit Gewebe bedeckt ist, so beträgt die Gewebeabdeckung und damit der Grad der Endothelialisierung 100%. In dem Fall, in dem die Einrichtung einen akustischen Resonator aufweist, wird der Grad der Endothelialisierung durch Messung der dem Implantat anhaftenden Masse bestimmt, während in dem Fall, in dem die Einrichtung einen elektrischen Resonator umfasst, der Grad der Endothelialisierung durch Messung der bedeckten Fläche bestimmt wird.

Direkt nach der Implantation ist kein Endothel auf der Oberfläche des Implantats vorhanden. Im Kaninchenmodell sind unbeschichtete Stents nach ca. 14 Tagen vollständig endothelialisiert, Stents mit antiproliferativen Medikamenten nach ca. 4 Wochen. Beim Menschen erfolgt das Einwachsen wesentlich langsamer, die Schätzung liegt bei einem Faktor 4 bis 6. Eine humane Studie, die das Einwachsen von Stents anhand von Autopsie-Präparaten untersucht, zeigt, dass die unbeschichteten Implantate nach 3 Wochen zu ca. einem Drittel endothelialisiert sind. (Anderson P. G., Bajaj R.K., Baxley W.A., Roubin G.S., "Vascular patholgy of balloon-expandable flexible coil stents in human", JACC 1992, 19, Seiten 372 bis 381).

Die aktuellen Empfehlungen der Konsensusgruppe "European Society of Cardiology Working Group on Thrombosis" für die Dauer einer dualen Antikoagulationstherapie sind 1 Monat für einen unbeschichteten Stent, mindestens 3 Monate für einen Sirolimus-, Everolimus- oder Tacrolimus-eluierenden Stent und 6 Monate für einen Paclitaxel-eluierenden Stent. Eine längere Dauer kann für ausgewählte Patienten mit einem geringem Blutungsrisiko in Betracht gezogen werden (Quelle: Lip G.Y., Huber K., Andreotti F., Arnesen H., Airaksinen K.J., Cuisset T., Kirchhof P., Marin F., "Management of antithrombotic therapy in atrial fibrillation patients presenting with acute coronary syndrome and/or undergoing percutaneous coronary interventionlstenting", Thromb Haemost 2010, 103, Seiten 13-28).

Als Endothel (lateinisch: endothelium) werden die zum Gefäßlumen hin gerichteten Zellen der innersten Wandschicht von Lymph- und Blutgefäßen (tunica intima) bezeichnet.

Insbesondere ist der akustische und/oder elektrische Resonator derart in und/oder am Implantat angeordnet, dass eine Änderung in der Masse und/oder der Fläche der Bedeckung der Oberfläche der Einrichtung durch Endothel eine entsprechende Änderung im elektrischen Ausgangssignal der Einrichtung bewirkt wird. Vorzugsweise sind der akustische Resonator und/oder der elektrische Resonator an der Innenseite (luminale Seite) des Implantats angeordnet, wobei besonders bevorzugt ein Abschnitt des jeweiligen Resonators an der Oberfläche frei liegt. Durch das Freiliegen der Abschnitte der jeweiligen Einrichtung wird bewirkt, dass das Endothel direkt auf der Oberfläche der Einrichtung wachsen kann und damit sehr genaue Ergebnisse hinsichtlich der Endothelialisierung erreicht werden können.

Besonders bevorzugt weist der akustische Resonator mindestens ein SAW-Element auf (SAW = Surface Acoustic Waves). Ein solches Element arbeitet auf der Basis akustischer Oberflächenwellen, d.h. Körperschall-Wellen, die sich planar auf der Oberfläche des Elements, d.h. in zwei Dimensionen ausbreiten). Ein solches SAW-Element nutzt die Abhängigkeit der Oberflächenwellengeschwindigkeit von der Beaufschlagung an Masse im Bereich der Oberfläche des SAW-Elements, d.h. der aufliegenden Masse an Körperzellen, wie Endothel, auf der Oberfläche des SAW-Elements. Besonders bevorzugt verwendet wird dabei ein SAW-Element, das auf der Basis von horizontalen Scherwellen (SH-SAW) arbeitet.

Bei diesem Ausführungsbeispiel wird die Eigenschaft der horizontalen Scherwellen, die eine Schwingungsebene parallel zur Oberfläche aufweisen, genutzt, dass diese nur gering in die Flüssigkeit (z.B. die Flüssigkeit in dem Gefäß) einkoppeln und daher die Wellenausbreitung durch die Flüssigkeit nur gering beeinflusst wird. Sogar eine "lose", auf der Oberfläche liegende Zelle, würde die Wellenausbreitung wenig stören. Nur an der Oberfläche festgewachsene Zellen, welche ja die gewünschte Endothelialisierung bilden, bewirken eine wesentliche Veränderung der Wellenausbreitung und damit eine Verringerung der Wellenausbreitungsgeschwindigkeit sowie folglich eine Verringerung der Resonanzfrequenz bzw. eine Erhöhung der Laufzeit. Gegenüber der Lösung nach dem oben erläuterten Stand der Technik mit einem Cantilever kann daher die Endothelialisierung durch das erfindungsgemäße Implantat viel genauer bestimmt werden.

In einem alternativen Ausführungsbeispiel weist der elektrische Resonator mindestens einen Kondensator und mindestens eine Spule auf, wobei der (ggf. aus parallel geschalteten Kondensatoren bestehende) Gesamt-Kondensator und die Spule in Reihe geschalteten sind und einen Schwingkreis ausbilden, dessen Resonanzfrequenz von der Gesamt-Kapazität des einen Kondensators bzw. der parallel geschalteten Vielzahl von Kondensatoren abhängt. Die sich verändernde Belegung des Implantats mit Körperzellen während der sich der Implantation anschließenden Heilung, d.h. das sich entsprechend ändernde Volumen des im Streufeld der Elektroden des mindestens einen Kondensators vorliegende Endothel, verringert die Streukapazität zwischen den Elektroden und erhöht somit die Resonanzfrequenz des elektrischen Resonators. Die Spule und der mindestens eine Kondensator können entweder an der luminalen Seite des Implantats befestigt oder einzeln oder gemeinsam als Teile des Implantats, z.B. die Stentstreben als Elektroden/Spulen, ausgebildet werden. Vorzugsweise können die Elektroden des Kondensators als Streifenelektroden auf einer geeigneten Folie ausgeführt werden, die ein Teil der luminalen Fläche des Implantats bedeckt.

Auch bei diesem Ausführungsbeispiel wird in vorteilhafter Weise die Eigenschaft des Kondensators ausgenutzt, dass das durch die Elektroden des Kondensators ausgebildete Streufeld sehr sensitiv auf den Abstand der Körperzellen von der Oberfläche des Implantats reagiert (proportional zu 1/(Abstand^2)). Je größer der Abstand einer Körperzelle zur Elektrodenebene (Oberfläche des Implantats) bzw. der Einrichtung, desto geringer ist sein Einfluss auf das Streufeld und somit auf die Resonanzfrequenz des Resonators. Folglich werden auch bei diesem Ausführungsbeispiel insbesondere die direkt an der Oberfläche des Implantats angelagerten Körperzellen, d.h. die Endothelialisierung, gemessen und somit ein entscheidender Vorteil gegenüber dem Stand der Technik erreicht.

Die Kapazität des Kondensators liegt vorzugsweise im Bereich zwischen 5 pF und 20 pF und die Induktivität der Spule vorzugsweise zwischen 200 nH und 500 nH. Die sich daraus ergebende Resonanzfrequenz des Schwingkreises liegt in dem bevorzugten Ausführungsbespiel somit im Bereich zwischen 50 MHz und 200 MHz.

Es ist weiter von Vorteil, wenn die Oberfläche der Einrichtung, insbesondere das SAW-Element des akustischen Resonators und/oder der Kondensator des elektrischen Resonators mindestens eine Beschichtung aufweist. Diese Beschichtung kann eine Passivierungsschicht sein und/oder eine Beschichtung, welche die gleichen oder zumindest ähnliche Bewuchseigenschaften wie der Grundkörper des Implantats aufweist. Insbesondere für den Kondensator eines elektrischen Resonators ist eine Beschichtung von Vorteil, welche isolierend ist und somit verhindert, dass die Elektroden des Kondensators kurzgeschlossen werden.

Geeignet für eine derartige Beschichtung ist insbesondere ein Polymermaterial, das über einen langen Zeitraum im Blutgefäß stabil ist und eine möglichst geringe Gewebereaktion hervorruft sowie nicht zur Bildung von Thromben neigt. Besonders geeignet ist eine dünne Schicht aus Parylene (z.B. Parylene-C), welche mittels eines Plasmaverfahrens aufgebracht wird. Auch andere inerte Polymere wie Polyurethane, Silikone, Teflon oder Acrylate sind geeignet.

Die Schichtdicke der Beschichtung über den Elektroden liegt vorzugsweise im Bereich von 1 μm bis 10 μm, wobei Schichten, die überwiegend aus Parylene bestehen, besonders bevorzugt Schichtdicken zwischen 1 μm und 3 μm aufweisen. Beschichtungen aus anderen Materialien liegen besonders bevorzugt im Bereich von 5 μm bis 10 μm.

Um eine drahtlose Abfrage der von der Einrichtung zur Messung der Endothelialisierung erzeugten Signale durch eine entsprechende Einrichtung zur Auswertung der Signale zu ermöglichen, weist die Einrichtung mindestens eine Antenne auf, welche mit dem akustischen Resonator und/oder dem elektrischen Resonator verbunden ist. Hierbei kann beispielsweise eine Stentstrebe als Antenne ausgebildet sein oder eine Antenne auf und/oder in den Grundkörper des Implantats auf- oder eingebracht sein. Die mindestens eine Antenne ist besonders bevorzugt an der Gefäßseite des Implantats, d.h. an der Außenseite bzw. der abluminalen Seite, angebracht, so dass diese nicht vom Implantat abgeschirmt wird (wie in einem Faradayschen Käfig). Alternativ könnten sie auch an beiden Enden in Richtung der Längsachse über das Implantat hinausragen.

Besonders bevorzugt enthalten Grundkörper des Implantats und Antenne das gleiche Material bzw. bestehen aus dem gleichen Material. Besonders geeignet sind Cobalt-Chrom-Stähle (L605, MP35N), aber auch medizinischer Edelstahl (316L) oder Nickel-Titan-Stähle (Nitinol). Gegebenenfalls kann eine schlechte Biokompatibilität eines Antennenmaterials durch Verwendung eines polymeren Coatings (z.B. mittels Parylene-C) verbessert werden.

Über diese mindestens eine Antenne wird der Mikrosensor von einem Abfragegerät aktiviert, welches mit mindestens einer entsprechenden Sendeantenne und mindestens einer entsprechenden Empfangsantenne ausgerüstet ist. Das Abfragegerät und/oder die in diesem integrierte oder mit diesem verbundene Recheneinheit berechnet auf der Basis der von dem Mikrosensor empfangenen Signale den Grad der Endothelialisierung und zeigt diesen an bzw. übermittelt diesen an eine mit der Recheneinheit verbundene Datenbank. Mit Hilfe der bereits in der Datenbank gespeicherten Daten zur Endothelialisierung während bereits zurückliegenden Zeiträumen kann dann der Verlauf der Endothelialisierung ermittelt und ggf. angezeigt werden.

In dem Ausführungsbeispiel, in dem ein akustischer Resonator verwendet wird, kann beispielsweise eine HF-Welle an einen akustischen Resonator mit Interdigital-Elektrode (IDT), die auf einem piezoelektrischen Material angeordnet ist, geleitet werden. Die IDT erzeugt hieraus ein akustisches Wellenpaket. Die Ausbreitung dieses Wellenpakets, das vorzugsweise als horizontale Scherwelle ausgebildet ist, in dem Implantat wird beobachtet und beispielsweise aus der Frequenzverschiebung des Resonators und/oder der Dämpfung des Signals und/oder der Verringerung der Güte des Resonators die Masse der Endothel-Schicht auf der Oberfläche des Implantats und damit der Grad der Endothelialisierung bestimmt. Hierfür kann ein Delay-Line-Sensor (Verzögerungsleitung) oder ein One-Port-Sensor in der Einrichtung verwendet werden.

Wird ein elektrischer Resonator genutzt, so wird beispielsweise mittels eines als Spektrumanalysator ausgebildeten Abfragegeräts die Resonanzfrequenz des Schwingkreises der Einrichtung bestimmt, welche von der Gesamtkapazität abhängt, die durch die Endothelialisierung veränderlich ist.

In einem Ausführungsbeispiel der Erfindung weist das Implantat zumindest auf einem Teil der Oberfläche seines Grundkörpers eine pharmazeutisch aktive Substanz auf.

Im Rahmen der vorliegenden Erfindung wird unter einer pharmazeutisch aktiven Substanz (oder therapeutisch aktive oder wirksame Substanz) ein pflanzlicher, tierischer oder synthetischer Wirkstoff (Medikament) oder ein Hormon verstanden, das in geeigneter Dosierung als Therapeutikum zur Beeinflussung von Zuständen oder Funktionen des Körpers, als Ersatz für natürlich vom menschlichen oder tierischen Körper erzeugte Wirkstoffe, wie Insulin, sowie zur Beseitigung oder zum Unschädlichmachen von Krankheitserregern, Tumoren, Krebszellen oder Körperfremdstoffen Einsatz findet. Die Freisetzung der Substanz in der Implantatumgebung hat einen positiven Effekt auf den Heilungsverlauf oder wirkt pathologischen Veränderungen des Gewebes in Folge des chirurgischen Eingriffs entgegen bzw. dient dem Unschädlichmachen von maladen Zellen in der Onkologie.

Derartige pharmazeutisch aktive Substanzen weisen beispielsweise eine antiinflammatorische und/oder antiproliferative und/oder spasmolytische Wirkung auf, wodurch beispielsweise Restenosen, Entzündungen oder (Gefäß-)Spasmen vermieden werden können. Derartige Substanzen können in besonders bevorzugten Ausführungsbeispielen aus einer oder mehreren Substanzen der Wirkstoffgruppe der Calciumkanalblokker, der Lipidregulatoren (wie beispielsweise Fibrate), der Immunsuppressiva, der Calcineurininhibitoren (wie beispielsweise Tacrolimus), der Antiflogistika (wie beispielsweise Cortison oder Dichlofenac), der Antiinflammatorica (wie beispielsweise Imidazole), der Antiallergika, der Oligonucleotide (wie beispielsweise dODN), der Östrogene (wie beispielsweise Genistein), der Endothelbildner (wie beispielsweise Fibrin), der Steroide, der Analgetika, der Antirheumatika der Proteine, der Hormone, der Insuline, der Zytostatika, der Peptide, der Vasodilatatoren (wie beispielsweise Sartane) und der antiproliferativ (proliferationshemmend) wirkenden Stoffe der Taxole oder Taxane, hier vorzugsweise Paclitaxel oder Sirolimus, bestehen, oder können der folgenden Liste entnommen werden: Cisplatin, Tirapazamine, Enzyme L-asparaginase, Methotrexate, 5-Fluorouracil, Azathioprine, Mitoxantrone, Cyclophosphamide, Methotrexate, Natalizumab, Adriamycin PFS, Adriamycin RDF, Alitretinoin, Altretamine, Aromasin, Azathioprine, Bicalutamide, Busulfan, Busulfex, Capecitabine, Casodex, Cyclophosphamide, Cytoxan, Doxorubicin, Exemestane, Femara, Finasteride, Gemtuzumab, Ozogamicin, Hexalen, Imuran, Letrozole, Mifeprex, Mifepristone, Myleran, Mylotarg, Neosar, Nolvadex, Panretin, Propecia, Proscar Rubex, Tamoxifen, Temodar, Temozolomide, Trelstar Depot, Triptorelin, Genasense (Fa.Genta), INGN201 (Fa.Introgen Therapeutics), SCH58500 (Fa.Schering-Plough), ONYX-015 (Fa.Onyx Pharmaceuticals), E1A-lipid complex (Fa.Targeted Genetics), TRAIL (Fa.Genentech/Immunex), GX01 (Fa.Gemin X Biotechnologies), Cyclosporin A, DPPE, PSC 833, Buthionine sulphoximine, Dexverapamil, Quinine, Verapamil, XR9576, Dexniguldipin, GF120918, Lobradimil, LY335979, MS209, R-101933, Gemtuzumabozogamicin, SGN-15, MCC-465, SB-408075, A5B7 antibody against CEA with carboxy peptidase A + mustard prodrug, Amifostine, Dexrazoxane, BB-10010, Transfer of MDR genes, BNP7787, Tirapazamine, Aplidine, Arsenic trioxide, BMS-247550, CHS828, CT 2584, Dolastatin-10, ET-743, Exisulind, Irofulven, KW-2189, Lovastatin, E7070, LU103793, LY355703, Pyrazoloacridine, TLK286, Apomine, CP-461, EP0906, FB642, FK317, FK866, Kahalalide F, LAF389, PNU-166196, RO 31-7453, Cetuximab (Erbitux), Trastuzumab (Herceptin), ABX-EGF, AP12009, EMD55900, EMD72000, ICR62, 2A11, CCI-779, ISIS-3521, Oblimersen (Genasense), OSI-774 (Tarceva), PS-341, R115777 (Zarnestra), STI571 (Gleevec), ZD1839 (Iressa), Bryostatin-1, Flavopiridol, GD0039, GEM231, Ilmofosine, ISIS-2503, ISIS-5132, L-778 123, PKC 412, SCH66336, SU-101, UCN-01, Bay 43-9006, BMS-214662, CI-1040, GW572016, LErafAON, LY-317615, Perifosine, Phenoxodiol, PKI 166, Swainsonine, 17-AAG, Decitabine, CI-994, Depsi-peptide, MG98, Phenylbutyrate, Phenylacetate, Sub-eroylanilidehydroxamic acid, Adp53, Antineoplastons, A10/AS2-1, OL(1)p53, p53, RPR/INGN-201, SCH 58500, HSV-TK VPC, tgDCC-E1A, INX3280, TK gene Pioglitazone, Troglitazone, BAY12-9566, BMS-275291, Clodronate, Marimastat, Prinomastat, MMI270, COL-3, CP-471,358, trans retinoic acid, Bexarotene, Pivaloyloxymethylbutyrate, 9-cis-retinoic acid, 13-cis RA, Fenretinide, ILX23-7553, TAC-101, Tazarotene, Bevacizumab, RhuMab-VEGF, HuMV833, Angiozyme, IMC-1C11, PI-88, SU5416, CP547,632, PNU-145156E, PTK/ZK 787, SU6668, ZD6474, Carboxyamidotriazole, GBC-590, Squalamine, Vitxain, ABT-510, CM101, ZD6126, Neovastat, Suramin, Thalidomide, IM862, TNP-470, Angiostatin, CC-5013, Combretastatin A4, Endostatin, Interleukin-12, Alemtuzumab, Edrecolomab, Epratuzumab HuM195, Oregovomab, Rituximab, Ch14.18, MDX-11, WX-G250, 3F8, H22xKI-4, ING-1, J591, KM871, Immunoconjugates antibody with toxin, BL22, Anti-Tac-PE38 (LMB-2), BB-10901, SS1-PE38, Denileukin diftitox (ONTAK), IL13-PE38QQR, TP-38, Allovectin-7, 105AD7, BEC2, TriGem, 1A7, 3H1, Vaccines, MDX-H210, G17DT, MDX-447, EMD 273063, IL-2/histamine, LAK, TIL, CTL, Bay 50-4798, MDX-010, OK-432, PSK, Ubenimex, GM-CSF, ONYX-015, NV1020, PV701, Reolysin, Celecoxib, Lyprinol, LY293111, Astrasentan, Melatonin, Taurolidine, Cyclosporin A, Verapamil, Tirapazamine Trastuzumab, Clodronate, trans retinoic acid, Edrecolomab, Rituximab, OK-432, Ubenimex, Melatonin, PSC 833, R115777, ZD1839, SCH 66336, Decitabine, HSV-TK, VPC, BAY12-9566, Marimastat, Prinomastat, Suramin, 105AD7, IL-2/histamine, Astrasentan.

Der Grundkörper eines erfindungsgemäßen Implantats kann vorzugsweise mindestens ein Element und/oder eine Verbindung aus der folgenden Gruppe enthaltend Metalle, Metalllegierungen, vorzugsweise Edel-stahl, CoCr-Stähle, Magnesiumlegierungen, Eisenlegie-rungen, Zinklegierungen, Manganlegierungen, Nitinol, Polymere aus der Klasse der bioabbaubaren Polymere, vorzugsweise Polymilchsäuren, Polygylcolsäuren, Polycaprolactone, Mischungen oder Copolymere daraus, Polymere aus der Klasse der biokompatiblen Polymeren, vorzugsweise UHMWPE und PEEK, enthalten. Hierbei wird ein Implantat gestaltet als Stent und bestehend aus einer biodegradierbaren Magnesiumlegierung, Eisenlegierung, Zinklegierung oder Manganlegierung als AMS (= absorbierbarer Metallstent) bezeichnet.

Die obige Aufgabe wird ferner durch ein System aus einem Katheter mit einem Ballon und einem Implantat gelöst, wobei das Implantat oben beschrieben ist und das Implantat auf dem, vorzugsweise gefalteten Ballon des Katheters angeordnet ist. Ein solches System eignet sich zum einfachen Einbringen des Implantats mit den oben angegebenen Vorteilen zur Behandlung in einen Organismus. Nach dem Platzieren des Katheters mit dem Implantat an der gewünschten Stelle wird während der Implantation der Ballon entfaltet und aufgeblasen und das Implantat hierdurch dilatiert. Wenn die erforderliche Dilatation, welche vorzugsweise auch zur Erweiterung des umgebenden Gefäßabschnitts dient, wird der Ballon wieder deflatiert und der Katheter kann entfernt werden, während das Implantat an der behandelten Stelle im Organismus verbleibt.

Das erfindungsgemäße Implantat und das erfindungsgemäße System werden nachfolgend in Beispielen anhand von Figuren erläutert. Dabei bilden alle beschriebenen und/oder bildlich dargestellten Merkmale den Gegenstand der Erfindung, auch unabhängig von ihrer Zusammenfassung in den Ansprüchen oder deren Rückbezüge.

Es zeigen schematisch:

Fig. 1      einen Abschnitt eines ersten Anführungsbeispiels eines erfindungsgenäßen Implantats in einer perspektivischen Ansicht von der Seite,

Fig. 2      eine Einrichtung, die in einem zweiten Ausfüh-rungsbeispiel eines erfindungsgemäßen Im-plantats zur Messung der Endothelialisierung dient, in einer Ansicht von oben,

Fig. 3      ein Teil einer Einrichtung, die in einem dritten Ausführungsbeispiel eines erfindungsgemäßen Implantats zur Messung der Endothelialisierung dient, in einer Ansicht von oben,

Fig. 4      ein System aus einem erfindungsgemäßen Implantat, einem Abfragegerät und einer Datenbank in einer Prinzipskizze und

Fig. 5      einen Längsschnitt durch die in Figur 2 dargestellte Einrichtung.

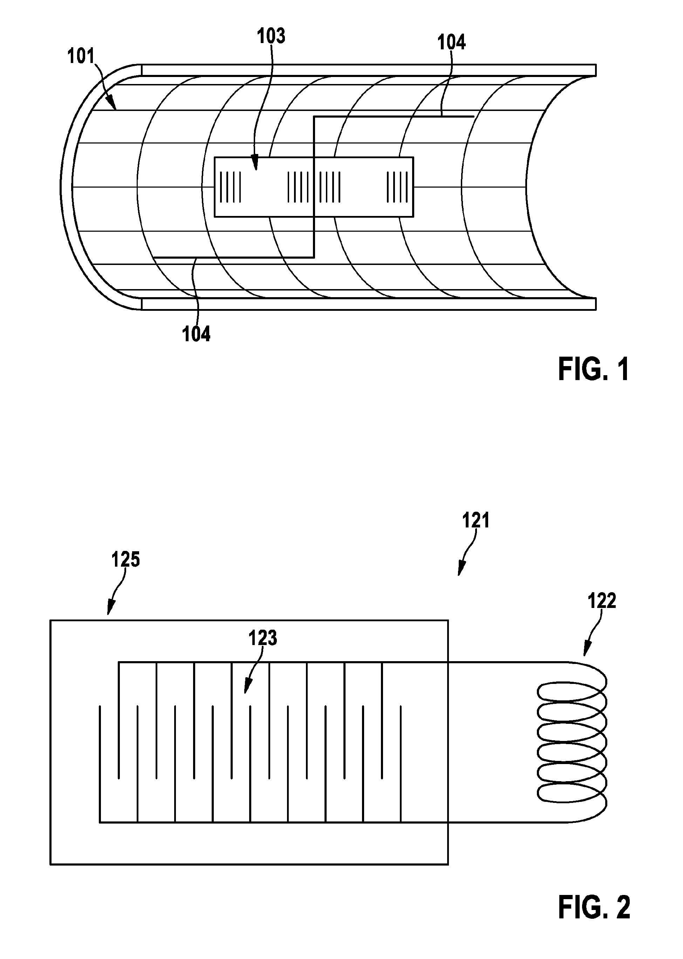

Figur 1 zeigt einen Abschnitt eines Grundkörpers 101 eines als Stent ausgebildeten Implantats, auf dessen luminaler Oberfläche ein SAW-Element 103 als Teil einer Einrichtung zur Messung der Endothelialisierung (im Folgenden kurz: Mikrosensor) angeordnet ist.

Das SAW-Element 103 ist mit einer ebenfalls zum Mikrosensor gehörenden Antenne 104 verbunden, welche eine drahtlose Abfrage der von dem SAW-Element 103 erzeugten Signale ermöglicht. Die Antenne 104 ist entweder am Grundkörper 100 befestigt oder als Teil des Grundkörpers 100 ausgebildet.

Das SAW-Element 103 bildet einen akustischen Resonator. Vorzugsweise wird ein sogenanntes Scherwellen-SAW-Element verwendet, das auf der Basis von horizontalen Schwerwellen arbeitet. Ein solches SAW-Element weist beispielsweise eine IDT auf, die auf einem piezoelektrischen Material angeordnet ist. Diese erzeugt eine Scherwelle, deren Ausbreitung im Implantat beispielsweise in einem Aufbau als Delay-Line-Sensor oder als One-Port-Sensors beobachtet und von dem Abfragegerät detektiert wird. Anhand der Verschiebung der Resonanzfrequenz, der Verringerung der Güte und/oder der Vergrößerung der Dämpfung des Resonators kann der Grad der Endothelialisierung bestimmt werden.

Auf der Oberfläche des SAW-Elements 103 kann eine nicht dargestellte Passivierungsschicht und/oder eine Beschichtung vorgesehen sein, welche gleiche oder zumindest ähnliche Bewuchseigenschaften wie die Oberfläche des Stent-Grundkörpers 100 aufweist.

Die Resonanzfrequenz des SAW-Elements 103 liegt beispielsweise im Bereich von 30 MHz bis 5 GHz, vorzugsweise wird eine Resonanzfrequenz von 400 MHz verwendet.

Die der Oberfläche des SAW-Elements 103 anhaftenden Zellen, d.h. die sich nach der Implantation anlagernden Endothelzellen, bewirken eine Vergrößerung der auf dem SAW-Element 103 aufliegenden Masse, welche die akustischen Eigenschaften des SAW-Elements 103 verändert. Dies führt bei einem Resonator beispielsweise zu einer Verringerung der Resonanzfrequenz und der Güte sowie eine Vergrößerung der Dämpfung. Diese Parameter können zur Bestimmung des Grades der Endothelialisierung des Implantats ausgewertet werden.

Entsprechend kann eine, außerhalb des mit dem erfindungsgemäßen Implantat behandelten menschlichen oder tierischen Körpers angeordnete Abfrageeinheit den Bewuchs des Implantats durch Körperzellen, d.h. die Endothelialisierung, qualitativ und quantitativ bestimmen.

Die Abfrageeinheit bestimmt beispielsweise die Resonanzfrequenz des SAW-Sensors mittels bekannter Verfahren und daraus die durch die Massebeladung verursachte Frequenzverschiebung Δf bezogen auf den unbeladenen Zustand. Aus dieser Frequenzverschiebung wird die Massebeladung Δm des SAW-Sensors und daraus der Grad der Endotheliasierung bestimmt mit

$$\Delta m = -k\,\frac{\Delta f}{f_0} \qquad (1),$$

wobei $f_0$ die Grundfrequenz des SAW-Resonators ohne Massebeladung und k eine Kalibrationskonstante der Sensoranordnung darstellen.

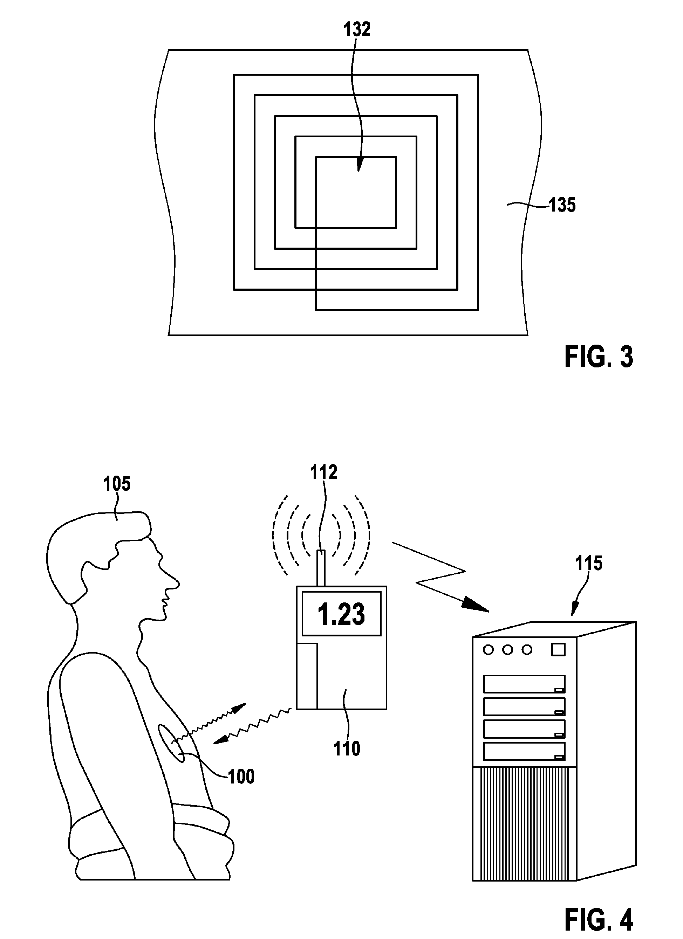

Über die Antenne 104 können die Signale vom SAW-Element 103 drahtlos abgefragt werden. Dieses ist in Figur 4 gezeigt. Im linken Bereich der Figur 4 ist ein in einen menschlichen Körper 105 implantierter Stent 100 veranschaulicht, an dem an der Innenseite ein SAW-Sensor angebracht ist, der mittels eines Abfragegeräts 110 abgefragt wird, das außerhalb des behandelten Körpers angeordnet ist. Auch das Abfragegerät 110 weist hierfür eine Antenne 112 auf, welche insbesondere als Sende-Empfangsantenne gestaltet ist. Das Abfragegerät 110 kann das SAW-Element 103 in regelmäßigen Abständen abfragen und die Signale empfangen, beispielsweise vom Patienten oder vom Arzt gesteuert. Die Abfrage kann auch automatisch ohne Mitwirkung des Patienten erfolgen, sobald sich dieser in der Nähe des Abfragegeräts 110 befindet. Die Abfrage wird in diesem Fall von dem Abfragegerät 110 autonom durchgeführt und gesteuert und kann z.B. einmal am Tag erfolgen.

Insbesondere dann, wenn die Abfrage mittels eines Abfragegeräts 110 eines Patienten durchgeführt wird, werden in einem Ausführungsbeispiel der vorliegenden Erfindung die von dem Mikrosensor ermittelten Daten oder die von einer Recheneinheit des Abfragegeräts 110 berechneten Daten, wie z.B. die Dicke der Endothel-Schicht auf dem Stent, vorzugsweise drahtlos an eine zentrale Datenbank übertragen, die Teil einer Recheneinheit 115 ist oder mit einer solchen verbunden ist. Die Daten werden in der Datenbank gespeichert, aufbereitet und dem behandelnden Arzt zur Verfügung gestellt. Die Darstellung für den Arzt kann beispielsweise in Form einer tabellarischen oder grafischen Verlaufsdarstellung der Dicke der auf dem Implantat angelagerten Endothel-Schicht erfolgen, so dass der Einwachsvorgang über einen bestimmten Zeitraum vorliegt, d.h. beispielsweise über ein Jahr. Die Recheneinheit 115 kann ferner eine Analyseeinrichtung aufweisen oder mit einer solchen verbunden sein, die eine automatische Warnung bzw. Benachrichtigung an den Arzt abgeben kann, wenn sich ein Zustand eingestellt hat, der eine Intervention erfordert.

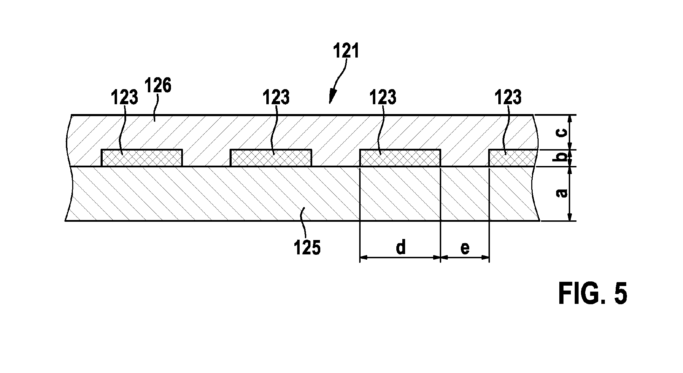

Alternativ oder zusätzlich zu dem SAW-Element 103 kann in dem Mikrosensor ein elektrischer Resonator 121 vorgesehen sein, der in den Figuren 2 und 5 gezeigt ist und eine Spule 122 und mehrere, einander gegenüberliegenden Elektroden 123 aufweist, die jeweils parallel geschaltete Kondensatoren ausbilden. Die Elektroden 123 können beispielsweise als Streifenelektroden auf einer Folie 125 dergestalt angeordnet sein, dass das elektrische Streufeld der Elektroden die nähere Umgebung des Mikrosensors durchdringt. Hierbei sind die Elektroden an der inneren (luminalen) Oberfläche des Stent-Grundkörpers 101 angeordnet, so dass die Elektroden bis auf eine isolierende Beschichtung frei liegen. Vorzugsweise besitzt das Material der Elektroden eine hohe elektrische Leitfähigkeit. Die Elektroden können daher aus einem Metall (Ag, Au, Cu, Al,....) oder einem leitfähigen Polymer bestehen. Beispielsweise kann eine solche Streifenelektrode eine Dicke b im Bereich von 10 $\mu$m bis 20 $\mu$m, eine Breite d von etwa 40 $\mu$m bis 60 $\mu$m, einen Abstand e der Streifen von etwa 50 $\mu$m und eine Länge abhängig von Stentdurchmesser aufweisen. Die Folie 125 hat beispielsweise eine Dicke a im Bereich von 100 $\mu$m bis 200 $\mu$m. Die genannten Abmessungen sind in Figur 5 eingezeichnet.

Die Elektroden 123 können ferner mit einer in Figur 5 gezeigten, vorzugsweise isolierenden Passivierungsschicht und/oder einer Beschichtung 126, die gleiche oder zumindest ähnliche Bewuchseigenschaften wie die Oberfläche des Implantatkörpers aufweist, versehen sein. Die Dicke c der Beschichtung 126 über den Elektroden 123 liegt im Bereich von 1 $\mu$m bis 10 $\mu$m.

Der elektrische Resonator 121 wird einschließlich Trägerfolie 125 auf den Stent-Grundkörper aufgebracht. Der Stent-Grundkörper wird dabei in dem Ausführungsbeispiel an seiner Innenseite (luminale Seite) mit der Trägerfolie 125 ausgekleidet.

Der Mikrosensor mit dem elektrischen Resonator 121 ist vorzugsweise an der luminalen Seite eines Stent-Grundkörpers angeordnet. Nachdem ein solches Implantat in den Körperhohlraum implantiert ist, so wird dieses mit Körperzellen bewachsen. Hierdurch verändert sich die Streukapazität zwischen den Elektroden 123, so dass sich die Resonanzfrequenz des Schwingkreises des elektrischen Resonators 121 ändert. Analog zum ersten Ausführungsbeispiel eines Implantats kann die Resonanzfrequenz dieses Schwingkreises und deren Änderung nach der Implantation mittels eines externen Abfragegeräts 110 ausgelesen werden. Hieraus kann die aktuelle Endothelialisierung des Implantats, deren Verlauf in der Vergangenheit sowie der Verlauf der Heilung abgeleitet werden. Es kann somit festgestellt werden, ob die Endothelialisierung sehr langsam verläuft und demnach eine erhöhte Gefahr für eine späte Thrombose (LST) besteht.

Anstelle der in Figur 2 gezeigten Spule 122 mit Streifenelektroden 123 kann auch die in Figur 3 dargestellte Planarspule 132 in dem elektrischen Resonator 121 verwendet werden. Die Planarspule 132 ist auf einer Folie 135 angeordnet.

Die Enden der in Figur 3 gezeigten Planarspule 132 sind miteinander verbunden. Der Schwingkreis ergibt sich in diesem Fall aus der Induktivität der Planarspule 132 und den Streukapazitäten zwischen den Leiterzügen der Planarspule 132. Diese jeweiligen Streukapazitäten verändern sich, wie oben beschrieben, durch die Endothel-Anlagerung auf der Oberfläche des mit der Planarspule 132 versehenen Implantats, wodurch sich ebenfalls die Resonanzfrequenz der Anordnung verändert.

Die momentane Dauer der Antikoagulationstherapie ist so bemessen, dass eine Endothelialisierung des Implantats bei möglichst allen Patienten gewährleistet ist. Diese Therapie wird aus Sicherheitsgründen über 6 bis 12 Monate wahrscheinlich länger als notwendig gegeben, was unnötige Kosten für das Gesundheitssystem verursacht.

Wenn die Endothelialisierung mittels eines erfindungsgemäßen Implantats ständig gemessen werden kann, ist eine bedarfsgerechtere und damit kürzere Antikoagulationstherapie, eine erhöhte Sicherheit durch eine Verlängerung dieser Therapie bei einzelnen Problempatienten und eine erhöhte subjektive Sicherheit der Patienten möglich.

Die erfindungsgemäße Lösung enthält nur ein passives Sensorelement, keine Batterie und keine Elektronik. Sie ist einfach, weist eine lange Lebensdauer auf und verändert die Abmessungen des Implantats nicht. Sie ermöglicht eine drahtlose, interventionsfreie Abfrage. Das erfindungsgemäße Implantat ist gegenüber dem Stand der Technik nur unwesentlich teurer, da der Mikrosensor kostengünstig herstellbar ist. Es ermöglicht die Erfassung des Endothelialisierungsverlaufs während der Heilung ohne Mitwirkung des Patienten und Arztes, eine maschinelle Auswertung der gewonnen Daten und eine automatische Benachrichtigung der beteiligten Personen im Bedarfsfall.

**Bezugszeichenliste**

| | |
|---|---|
| 100 | Stent |
| 101 | Grundkörper des Implantats 100 |
| 103 | SAW-Element |
| 104 | Antenne |
| 105 | menschlicher Körper |
| 110 | Abfragegerät |
| 112 | Antenne |
| 115 | Recheneinheit |
| 121 | elektrischer Resonator |
| 122 | Spule |
| 123 | Elektrode |
| 125 | Folie |
| 126 | Beschichtung |
| 132 | Planarspule |
| 135 | Folie |
| a | Dicke der Folie 125 |
| b | Dicke der Elektrode 123 |
| c | Dicke der Beschichtung 126 oberhalb der Elektrode 123 |
| d | Breite der Elektrode 123 |
| e | Breite des Zwischenraums zwischen den Elektroden 123 |

**Patentansprüche**

1. Implantat (100) mit einem vorzugsweise hohlzylinderförmigen Grundkörper (101) und mit einer Einrichtung, die zur Messung des Grads der Endothelialisierung dient und die an und/oder im Grundkörper angeordnet ist, wobei die Einrichtung einen akustischen Resonator (103) und/oder einen elektrischen Resonator (121) aufweist.

2. Implantat nach Anspruch 1, **dadurch gekennzeichnet, dass** der akustische Resonator (103) und/oder der elektrische Resonator (121) derart angeordnet ist/sind, dass eine Änderung in der Masse und/oder der Fläche der Bedeckung der Oberfläche der Einrichtung durch Endothel eine entsprechende Änderung im elektrischen Ausgangssignal der Einrichtung bewirkt.

3. Implantat nach einem der vorhergehenden Ansprüche, **dadurch gekennzeichnet, dass** der akustische Resonator (103) mindestens ein SAW-Element aufweist.

4. Implantat nach einem der vorhergehenden Ansprüche, **dadurch gekennzeichnet, dass** der elektrische Resonator (121) mindestens einen Kondensator und mindestens eine Spule (122) aufweist.

5. Implantat nach einem der vorhergehenden Ansprüche, **dadurch gekennzeichnet, dass** die Oberfläche der Einrichtung mindestens eine Beschichtung aufweist.

6. Implantat nach einem der vorhergehenden Ansprüche, **dadurch gekennzeichnet, dass** die Einrichtung eine Antenne aufweist.

7. Implantat nach einem der vorhergehenden Ansprüche, **dadurch gekennzeichnet, dass** das Implantat zumindest auf einem Teil der Oberfläche seines Grundkörpers eine pharmazeutisch aktive Substanz aufweist.

8. System aus einem Katheter mit einem Ballon und einem Implantat nach einem der vorhergehenden Ansprüche, **dadurch gekennzeichnet, dass** das Implantat auf dem Ballon des Katheters angeordnet ist.

FIG. 1

FIG. 2

**132**

**135**

**FIG. 3**

**105**

**112**

**1.23**

**110**

**115**

**100**

**FIG. 4**

FIG. 5

Europäisches
Patentamt

European
Patent Office

Office européen
des brevets

**EUROPÄISCHER RECHERCHENBERICHT**

Nummer der Anmeldung

EP 12 18 8615

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2009/062900 A1 (LAL RATNESH [US] ET AL) 5. März 2009 (2009-03-05) * Absätze [0034] - [0081]; Abbildung 1 * ----- | 1,4-8 | INV. A61F2/82 A61B5/00 |
| X | WO 01/12092 A1 (GOODRICH CO B F [US]) 22. Februar 2001 (2001-02-22) * Seite 11, Zeile 26 - Seite 12, Zeile 29; Abbildung 6a * * Seite 9, Zeile 11 - Zeile 16 * ----- | 1-5 | |
| X | WO 2010/019773 A2 (PROTEUS BIOMEDICAL INC [US]; ROBERTSON TIMOTHY [US]) 18. Februar 2010 (2010-02-18) * Seite 6, Zeile 12 - Seite 7, Zeile 14; Abbildung 1 * ----- | 1,2,4 | |
| X | US 2005/277839 A1 (ALDERMAN RICHARD A [US] ET AL) 15. Dezember 2005 (2005-12-15) * Absatz [0041] - Absatz [0044]; Abbildungen 3,4 * ----- | 1-3,6 | |
| | | | **RECHERCHIERTE SACHGEBIETE (IPC)** A61F A61B |

Der vorliegende Recherchenbericht wurde für alle Patentansprüche erstellt

| Recherchenort | Abschlußdatum der Recherche | Prüfer |
|---|---|---|
| München | 13. Februar 2013 | Skorovs, Peteris |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument
...........................................................................................
& : Mitglied der gleichen Patentfamilie, übereinstimmendes
    Dokument

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**

EP 12 18 8615

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

13-02-2013

| Im Recherchenbericht angeführtes Patentdokument | Datum der Veröffentlichung | Mitglied(er) der Patentfamilie | Datum der Veröffentlichung |
|---|---|---|---|
| US 2009062900 A1 | 05-03-2009 | KEINE | |
| WO 0112092 A1 | 22-02-2001 | AU 6625600 A<br>WO 0112092 A1 | 13-03-2001<br>22-02-2001 |
| WO 2010019773 A2 | 18-02-2010 | KEINE | |
| US 2005277839 A1 | 15-12-2005 | KEINE | |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **K. M MUSICK et al.** Sensor to detect endothelialization on an active coronary stent. *BioMedical Engineering,* 2010, vol. 9, 67
- **ANDERSON P. G. ; BAJAJ R.K. ; BAXLEY W.A. ; ROUBIN G.S.** Vascular patholgy of balloon-expandable flexible coil stents in human. *JACC,* 1992, vol. 19, 372-381
- **LIP G.Y. ; HUBER K. ; ANDREOTTI F. ; ARNESEN H. ; AIRAKSINEN K.J. ; CUISSET T. ; KIRCHHOF P. ; MARIN F.** Management of antithrombotic therapy in atrial fibrillation patients presenting with acute coronary syndrome and/or undergoing percutaneous coronary interventionlstenting. *Thromb Haemost,* 2010, vol. 103, 13-28